(19) Europäisches Patentamt
European Patent Office
Office européen des brevets

(11) Veröffentlichungsnummer: **0 034 365**
**B1**

(12) # EUROPÄISCHE PATENTSCHRIFT

(45) Veröffentlichungstag der Patentschrift: **29.06.83**

(51) Int. Cl.³: **C 07 J 7/00,** C 07 J 41/00

(21) Anmeldenummer: **81101115.4**

(22) Anmeldetag: **17.02.81**

(54) **Verfahren zur Herstellung von 3,20-Dioxo-pregn-4-en und/oder 3,20-Dioxo-pregna-1,4-dien.**

(30) Priorität: **19.02.80 US 122396**

(43) Veröffentlichungstag der Anmeldung: **26.08.81 Patentblatt 81/34**

(45) Bekanntmachung des Hinweises auf die Patenterteilung: **29.06.83 Patentblatt 83/26**

(84) Benannte Vertragsstaaten: **AT BE CH DE FR GB IT LI LU NL SE**

(56) Entgegenhaltungen:

**JOURNAL OF THE AMERICAN CHEMICAL SOCIETY, Band 91, Nr. 6, 12. März 1969, Seiten 1429-1432, Washington D.C., U.S.A., E.J. COREY et al.: «A new method for the oxidation of primary amines to ketones»**

(73) Patentinhaber: **HENKEL CORPORATION, 4620 West 77th Street, Minneapolis Minnesota 55435 (US)**

(72) Erfinder: **Krbechek, Leroy O., 2041 Orkla Drive, Minneapolis Minnesota 55427 (US)**
Erfinder: **Clark, Jim P., Route 2, Box 106 K, Forest Lake Minnesota 55025 (US)**
Erfinder: **Spitzner, Ernest B., 5315 Dupont Avenue South, Minneapolis Minnesota 55419 (US)**

(74) Vertreter: **Fues, Johann Friedrich, Dr. et al, Deichmannhaus am Hauptbahnhof, D-5000 Köln 1 (DE)**

**EP 0 034 365 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von 3,20-Dioxo-pregn-4-en und/oder 3,20-Dioxo-pregna-1,4-dien

Die Erfindung betrifft eine wichtige Teilstufe bei der Herstellung von Progesteron und/oder Dehydroprogesteron, insbesondere ausgehend von Steroidverbindungen, die in 17-Stellung den α -Propionsäurerest aufweisen.

In der offengelegten Europäischen Patentanmeldung 004 913 ist u.a. ein Verfahren zur Herstellung von 17-C-Steroid-α-Propionsäureverbindungen — insbesondere zur Herstellung von 3-Oxo-pregna-4--en-20-carbonsäure($\Delta^4$-BNC) und/oder 3-Oxo--pregna-1,4-dien-20-carbonsäure($\Delta^{1,4}$-BNC) — durch mikrobiellen Seitenabbau an 17-C-Seitenketten-Steroidsubstraten beschrieben. Sollen diese Verbindungen als Ausgangsmaterial zur Gewinnung von Progesteron bzw. Dehydroprogesteron eingesetzt werden, so bedarf es des Abbaus der in 20-Stellung substituierten Carboxylgruppe und Ausbildung einer 20-Oxogruppe. Diese Umwandlung gelingt in einem mehrstufigen Verfahren. Die Erfindung betrifft einen wichtigen Teilaspekt hieraus.

Gegenstand der Erfindung ist dementsprechend ein Verfahren zur Herstellung von 3,20-Dioxo--pregn-4-en und/oder 3,20-Dioxo-pregna-1,4-dien, wobei dieses Verfahren dadurch gekennzeichnet ist, dass man 20-Amino-3-oxo-pregn-4-en und/oder 20-Amino-3-oxo-pregna-1,4-dien mit einem 3,5-disubstituierten o-Chinon zum entsprechenden Steroid-20-Anil umsetzt und dieses hydrolysiert. Bevorzugt werden entsprechende o-Benzochinonverbindungen eingesetzt.

Die erfindungsgemäss als Ausgangsmaterial einzusetzenden 20-Amine und ihre vorgängigen Reaktionsstufen sind aus den beiden folgenden Formelbildern A und B zu entnehmen:

A

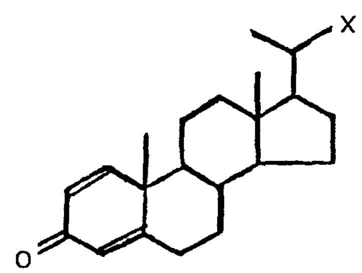

B

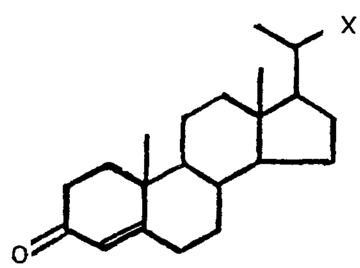

A = $\Delta^4$-BNC, B = $\Delta^{1,4}$-BNC.

Bedeutet in diesen Formelbildern X die Carboxylgruppe -COOH, so ist die Ausgangssäure dargestellt. Diese Säure kann — vorzugsweise durch Behandlung mit Thionylchlorid — zum Säurehalogenid, insbesondere Säurechlorid, umgewandelt werden, wobei dann X die Bedeutung von -COCl hat. Das Säurechlorid lässt sich insbesondere mit Ammoniak zum Säureamid umwandeln, wobei X die Bedeutung von $-CONH_2$ annimmt. Das Säureamid lässt sich auf dem Wege über das Bromamid — X hat hier die Bedeutung von -CONHBr — zum Isocyanat (X = -NCO) umlagern, woraufhin durch Addition von Alkoholen das Carbamat gebildet wird — X bedeutet hier den Rest $-NH(CO_2)R$, worin R den Kohlenwasserstoffrest des eingesetzten Alkohols darstellt. Abschliessend kann das Carbamat zum Amin hydrolysiert werden, in dem X die Bedeutung $-NH_2$ hat. Einzelheiten zu diesen Verfahrensstufen finden sich in den parallelen Schutzrechtsanmeldungen: Europäische Patentanmeldung 81 100 145.2 sowie parallele Europäische Patentanmeldungen 81 101 119.6/34 368 («Verfahren zur Umwandlung von Steroid-20-Carbonsäureamiden in die entsprechenden Steroid-20--Carbamate», U.S.Priorität vom 19. Februar 1980, Aktenzeichen: 122 395, Case 4166) 81 101 114.7/34 364 («Neue 20-substituierte 3-Oxo-pregna-4-en--steoridverbindungen und Verfahren zu ihrer Herstellung», U.S.Priorität vom 19. Februar 1980, Aktenzeichen: 122 397, Case 4168) 81 101 117.0/34 366 («Verfahren zur Herstellung von Steroid-20--Carbamatverbindungen», U.S.Priorität vom 19. Februar 1980, Aktenzeichen: 122 398, Case 4174) 81 101 113.9/34 363 («Neue Steroidverbindung mit einer 20-Amino-Funktion und Verfahren zu ihrer Herstellung», U.S.Prioritäten vom 19. Februar 1980, Aktenzeichen: 122 397 und 122 321, Cases 4168/4183) der gleichen Anmelderin.

Ausgangsmaterial für das hier beanspruchte Verfahren der Erfindung sind die 20-Aminoverbindungen, in denen X in den Formelbildern A bzw. B die Bedeutung von $-NH_2$ hat.

Die 20-Aminoverbindung wird als freies Amin der Umsetzung der ersten erfindungsgemässen Stufe zugeführt. Liegt die Aminoverbindung als Salz vor, beispielsweise als Hydrochlorid, als Bisulfat oder andere vergleichbare salzartige Verbindung, so ist es notwendig, das Aminsalz mit entsprechend starken Basen zu behandeln, um die 20-Aminoverbindung freizusetzen. Geeignete Basen sind beispielsweise NaOH, KOH oder Natrium-methoxid.

In der ersten Verfahrensstufe wird das freie Amin mit einem 3,5-disubstituierten o-Chinon zur entsprechenden Steroid-Anil-Verbindung umgesetzt. Die o-Chinonverbindung wird dabei wenigstens in äquivalenten Mengen, bezogen auf eingesetztes Steroidamin verwendet. Es kann angenommen werden, dass durch eine Reihe von Reaktionsschritten der Aminstickstoff in 20-Stellung der Steroidstruktur eine Doppelbindung mit der Chinonringstruktur in o-Stellung zur Carbonylgruppe des Chinons ausbildet. Dieses Zwischenprodukt wandelt sich dann spontan zu der im folgenden gezeigten Anilstruktur

gemäss C und D um.

Die Anilverbindung C ist das vorwiegend anfallende Reaktionsprodukt, wobei jedoch Anilverbindungen der Struktur D in geringerer Menge vorliegen können.

In diesen Formeln C und D bedeutet St den Rest des Steroidringsystems beginnend mit C-17.

C

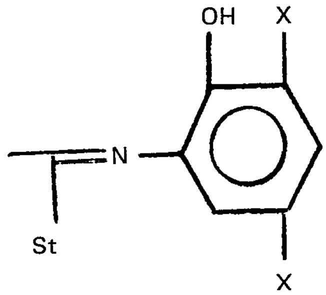

D

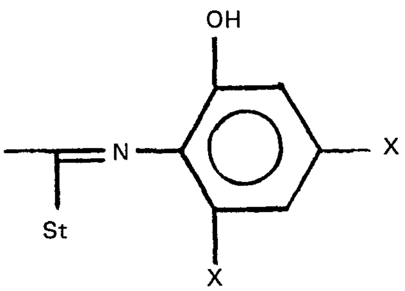

Die in den Formelbildern C und D gezeigten Substituenten X in 3,5-Stellung können beliebige Reste sein, die die Ausbildung der Anilstruktur zulassen. Bevorzugt bedeutet X Alkylreste, und zwar insbesondere mit bis zu 10 C-Atomen, vorzugsweise mit 1 bis 5 C-Atomen. In einer besonders bevorzugten Ausführungsform der Erfindung ist X der tert.-Butylrest.

Zur Ausbildung der Steroid-Anil-Verbindung wird das 3,5-disubstituierte Chinon wenigstens in äquivalenter Menge, bezogen auf eingesetztes Steroid-20--Amin verwendet, wobei es bevorzugt sein kann, etwa äquivalente Mengen der Reaktionspartner bzw. nur einen leichten Überschuss der o-Chinonverbindung einzusetzen.

Bevorzugt ist es weiterhin, die Umsetzung in inerter Atmosphäre, vorzugsweise unter Stickstoff durchzuführen. Die Reaktionstemperatur zur Anilbildung liegt zweckmässigerweise im Bereich von etwa −10°C bis etwa 35°C.

Die Umsetzung des Steroid-20-Amins mit dem 3,5-disubstituierten o-Chinon wird vorzugsweise in Lösungsmitteln durchgeführt. Alkohole, insbesondere Alkanole, sind geeignete Lösungsmittel. In Betracht kommen beispielsweise Methanol, Äthanol, Isopropanol oder Butanol. Bevorzugte Lösungsmittel sind Methanol oder Äthanol und insbesondere Methanol.

Die gebildete Steroid-Anil-Verbindung bzw. das Gemisch der hier in Betracht kommenden Anilverbindungen kann isoliert werden. Diese Steroid-Anil-Verbindungen bilden dementsprechend als solche einen weiteren Gegenstand der vorliegenden Erfindung. In einer bevorzugten Ausführungsform der Erfindung wird jedoch von einer Isolierung der Steroid-Anil-Verbindungen Abstand genommen. Statt dessen wird das Reaktionsgemisch — vorzugsweise in einem Zuge — der weiteren Umsetzung durch Hydrolyse unterworfen.

Die Hydrolysestufe wird zweckmässigerweise im sauren, insbesondere im vergleichsweise schwachsauren pH-Bereich durchgeführt. Bevorzugt liegt der pH-Wert des Reaktionsmediums bei der Hydrolyse im Bereich von etwa 2,5 bis etwa 4,5. Zweckmässigerweise wird dabei in einem Puffersystem gearbeitet. Ein geeignetes Puffersystem ist beispielsweise Essigsäure/Amonacetat, das in wässriger Mischung eingesetzt werden kann und dann gleichzeitig das Wasser zum Hydrolyseschritt zur Verfügung stellt. In der besonders bevorzugten Ausführungsform des erfindungsgemässen Verfahrens liegt der pH-Wert während des Hydrolyseschrittes im Bereich von etwa 3,0 bis 4,0.

Für die Hydrolyse ist die Gegenwart wenigstens äquivalenter Wassermengen erforderlich, die — wie angegeben — über das Puffersystem in die Reaktionsmischung eingebracht werden können. Es hat sich gezeigt, dass eine basische Hydrolyse prinzipiell zwar durchgeführt werden kann, jedoch Ausbeute und Reinheit des Verfahrensprodukts beeinträchtigen.

Die Verfahrenstemperatur während der Hydrolysestufe liegt zweckmässigerweise im Bereich von etwa −10°C bis etwa 100°C, vorzugsweise im Bereich oberhalb 0°C bis etwa 100°C. Bevorzugt wird auch hier unter Ausschluss von Luftsauerstoff gearbeitet.

In einer weiteren Ausführungsform der Erfindung werden im erfindungsgemässen Verfahren und insbesondere in den abschliessenden Stufen des erfindungsgemässen Verfahrens nicht-steroide Carbonylverbindungen mitverwendet, die eine Ketogruppe oder eine Aldehydgruppe aufweisen.

Es hat sich gezeigt, dass das erfindungsgemäss angestrebte Verfahrensprodukt in erhöhten Ausbeuten und grösserer Reinheit erhalten werden kann, wenn solche Carbonylverbindungen im Reaktionsgemisch mitverwendet werden. Diese Komponenten können vor oder während der Hydrolysestufe oder vor dem Abziehen des Lösungsmittels zugesetzt werden. Schwierigkeiten, die bei der Hydrolyse und/oder beim Abziehen des Lösungsmittels auftreten können, werden durch den Zusatz solcher Carbonylverbindungen vermindert. Die Carbonylverbindung kann zweckmässigerweise in Mengen von etwa 0,01 Äquivalenten bis etwa 10 Äquivalenten, bezogen auf die Steroidverbindung, eingesetzt werden.

Als Carbonylverbindungen kommen beispielsweise entsprechende aliphatische oder cycloaliphatische Verbindungen mit bis zu 10 C-Atomen in Betracht, die auch olefinisch ungesättigt sein können. Geeignete Aldehyde und Ketone, die im Rahmen der Erfindung verwendet werden können, sind beispielsweise Cyclohexanon, Cyclohexenon, Methylethylketon, Methylisobutylketon, Aceton, n-Butyraldehyd und 2-Ethylhexyaldehyd. Aber auch andere Ke-

toverbindungen, die Lävulinsäure und Brenztraubensäure, bzw. ihre Ester, reduzieren einen eventuellen Verlust an Endprodukt während des Abtreibens des Lösungsmittels und/oder einer unerwünschten Zersetzung während der Hydrolyse.

Steroidverbindungen mit einer Säurefunktion in der Seitenkette und chemische Reaktionen an solchen Verbindungen sind im Stand der Technik mehrfach beschrieben. Hinweise auf die erfindungsgemässe Reaktion finden sich darin jedoch nicht. Aus dem Stand der Technik seien genannt: US-PS 4 088 760; JACS, Band 70, Nr. 3, S. 887; US-PSen 2 566 336 und 2 731 461; GB-PS 1 043 018; Chemische Berichte, Jahrgang 88 (1955), S. 883-984; US-PSen 2 752 369 und 3 519 658.

Aminsteroidverbindungen werden geschildert in J. Am. Chem. Soc. 1956, S. 524 ff. sowie in Tetrahedron Letters, Nr. 18 (1964), S. 1053-1061. Verwiesen wird schliesslich auf Hevetica Chimica Acta, Band 32 (1949), Teil V, Nr. 233, S. 1764 sowie a.a.O. Teil V, Nr. 232, S. 1758 sowie a.a.O. Teil VI, Nr. 255, S. 1922.

*Beispiel*

0,5 g 20-Amino-3-oxo-pregn-4-en-hydrochlorid werden mit 2,85 ml 0,5molarem Natriummethoxid und 2,2 ml Methanol zur Umsetzung gebracht. Das Reaktionsgefäss wird 3mal evakuiert und unter Stickstoff gesetzt. Die Lösung wird dann in einem Eisbad gekühlt. Es werden 0,32 g (1,1 Äquivalente) 3,5-Di-tert.-butyl-o-benzochinon mit 5,0 ml Methanol in das Reaktionsgemisch eingetragen.

Das Reaktionssystem wird erneut 3mal evakuiert und wiederum unter Stickstoff gesetzt. Anschliessend wird die Mischung unter Eiskühlung für den Zeitraum von etwa einer Stunde gerührt. Die Dünnschicht-Chromatographie zeigt danach, dass sich das gewünschte Imin ausgebildet hat und nur noch sehr wenig restliches Amin vorliegt.

Es werden jetzt 50 ml eines 1:1 (Volumenteile) Gemisches von Tetrahydrofuran und Methanol zugegeben, das zuvor unter einem Stickstoffstrom mit Stickstoff entgast worden ist. Das Reaktionssystem wird erneut 3mal evakuiert und mit Stickstoff gespült. Anschliessend wird das Eisbad entfernt. Es werden 25 ml eines 0,1molaren Puffers von Ammoniumacetat/Essigsäure mit einem pH von 3,8 zugesetzt. Der Puffer ist zuvor ebenfalls mit Stickstoff entgast worden und wird unter einem Stickstoffstrom zugesetzt.

Das System wird wiederum 3mal evakuiert und erneut unter Stickstoff gesetzt. Anschliessend wird die Reaktionsmischung für etwa 0,5 Stunden am Rückfluss gekocht. Danach zeigt die Dünnschicht-Chromatographie keine Iminreste, jedoch beträchtliche Mengen an Progesteron (3,20-Dioxo-pregn-4--en). Die Lösung wird dann auf Raumtemperatur gekühlt und unter Verwendung von 250 ml des zuvor beschriebenen Gemisches von Tetrahydrofuran und Methanol sowie 175 ml der Pufferlösung auf ein Reaktionsvolumen von 500 ml verdünnt. Anschliessend wird die Lösung erneut mit Stickstoff entgast und auf Raumtemperatur im Eisbad gekühlt und wiederum mit Stickstoff entgast. Progesteron wird schliesslich in einer Ausbeute von 94% der Theorie gewonnen.

Der Zusatz von Carbonylverbindungen der angegebenen Art in einer Menge von 0,1 Äquivalent, bezogen auf eingesetzte Steroidverbindung, vor der Hydrolyse oder während der Hydrolyse ermöglicht eine Verbesserung der Verfahrensausbeute.

Wird das Ausgangsmaterial 20-Amino-3-oxo--pregna-1,4-dien im gleichen Verfahren eingesetzt, so wird das 3,20-Dioxo-pregna-1,4-dien in vergleichbaren Ausbeuten erhalten.

**Patentansprüche**

1. Verfahren zur Herstellung von 3,20-Dioxo--pregn-4-en und/oder 3,20-Dioxo-pregna-1,4-dien, dadurch gekennzeichnet, dass man 20-Amino-3--oxo-pregn-4-en und/oder 20-Amino-3-oxo-pregna--1,4-dien in Form des freien Amins mit einem 3,5-disubstituierten o-Chinon zum entsprechenden Steroid-20-anil umsetzt und dieses hydrolysiert.

2. Verfahren nach Anspruch 1, dadurch gekennzeichnet, dass die Hydrolyse im pH-Bereich von etwa 2,5 bis 4,5 und insbesondere im Bereich von etwa 3,0 bis 4,0 durchgeführt wird, wobei man vorzugsweise in einem Puffersystem — z.B. einem Essigsäure-Amonacetat-Puffer — arbeitet.

3. Verfahren nach Ansprüchen 1 und 2, dadurch gekennzeichnet, dass dem Reaktionsgemisch vor der Isolierung des Produkts aus der Hydrolysestufe eine nicht-steroide Carbonylverbindung zugesetzt wird, wobei bevorzugt Ketone oder Aldehyde eingesetzt werden und entsprechende aliphatische oder cycloaliphatische Verbindungen mit bis zu 10 C-Atomen, die auch olefinisch ungesättigt sein können, bevorzugt sind.

4. Verfahren nach Ansprüchen 1 bis 3, dadurch gekennzeichnet, dass die Menge der zugesetzten Carbonylverbindung im Bereich von 0,01 bis 10 Äquivalente pro Äquivalent Steroidverbindung beträgt.

5. Verfahren nach Ansprüchen 1 bis 4, dadurch gekennzeichnet, dass die nicht-steroide Carbonylverbindung dem Reaktionsgemisch vor oder während des Hydrolyseschrittes zugesetzt wird.

6. Verfahren nach Ansprüchen 1 bis 5, dadurch gekennzeichnet, dass mit 3,5-dialkyl-substituierten o-Chinon-Verbindungen gearbeitet wird, deren Alkylsubstituenten vorzugsweise jeweils bis zu 10, insbesondere bis zu 5 C-Atome aufweisen.

7. Verfahren nach Ansprüchen 1 bis 6, dadurch gekennzeichnet, dass die Ausbildung der Steroid-Anilverbindungen bei Temperaturen von etwa −10°C bis 35°C, vorzugsweise in Lösungsmitteln, wie Alkanolen, und zweckmässig unter Inertgas erfolgt.

8. Verfahren nach Ansprüchen 1 bis 7, dadurch gekennzeichnet, dass die Hydrolyse bei Temperaturen von −10°C bis 100°C und vorzugsweise ebenfalls unter Ausschluss von Luftsauerstoff durchgeführt wird.

9. Verfahren nach Ansprüchen 1 bis 8, dadurch gekennzeichnet, dass mit 3,5-Di-tert.-butyl-o-benzochinon gearbeitet wird.

10. Verfahren nach Ansprüchen 1 bis 9, dadurch gekennzeichnet, dass die Reaktion in einem Zug ohne Isolierung der intermediär entstehenden Steroid-Anil-Verbindungen durchgeführt wird.

**Claims**

1. A process for preparing 3,20-dioxo-pregn-4--ene and/or 3,20-dioxo-pregna-1,4-diene, characterized by reacting 20-amino-3-oxo-pregn-4-ene and/or 20-amino-3-oxo-pregna-1,4-diene in the free amine form(s) with a 3,5-disubstituted o-quinone to give the respective steroid-20-anil and hydrolyzing same.

2. The process according to claim 1, characterized in that the hydrolysis is carried out in a pH range from about 2.5 to 4.5, and more particularly from about 3.0 to 4.0, the process being preferably carried out in a buffer system, e.g. an acetic acid/ammonium acetate buffer.

3. The process according to claims 1 and 2, characterized in that prior to the isolation of the product from the hydrolysis step a non-steroid carbonyl compound is added to the reaction mixture, ketones and aldehydes being preferably employed therefor, and respective aliphatic or cycloaliphatic compounds having up to 10 carbon atoms, which compounds may also be olefinically unsaturated, being preferred.

4. The process according to claims 1 to 3, characterized in that the amount of the added carbonyl compound is in the range of from 0.1 to 10 equivalents per equivalent of the steroid compound.

5. The process according to claims 1 to 4, characterized in that the non-seroid carbonyl compound is added to the reaction mixture prior to or during the hydrolysis step.

6. The process according to claims 1 to 5, characterized in that 3,5-dialkyl-substituted o-quinone compounds are employed, the alkyl substituents of which preferably have up to 10, more specifically up to 5, carbon atoms.

7. The process according to claims 1 to 6, characterized in that the formation of the steroid-anil compounds is effected at temperatures of about −10°C to 35°C, preferably in solvents such as alkanols, and conveniently under an inert gas.

8. The process according to claims 1 to 7, characterized in that the hydrolysis is effected at temperatures of about −10°C to 100°C, and preferably also with the oxygen of the air being excluded.

9. The process accoding to claims 1 to 8, characterized in that 3,5-di-tert-butyl-o-benzoquinone is employed.

10. The process according to claims 1 to 9, characterized in that the reaction is carried out at one pull without isolating the steroid-anil compounds being intermediately formed.

**Revendications**

1. Procédé pour la fabrication de 3,20-dioxo--prégna-4-ène et/ou de 3,20-dioxo-prégna-1,4-diène, caractérisé en ce que l'on fait réagir le 20-amino-3-oxo-prégna-4-ène et/ou le 20-amino-3-oxo--prĕgna-1,4-diène sous forme de l'amine libre avec une o-quinone 3,5-disubstituée en le stéroïde-20--anile et on hydrolyse celui-ci.

2. Procédé selon la revendication 1, caractérisé en ce que l'on effectue l'hydrolyse dans un domaine de pH d'environ 2,5 à 4,5, et en particulier dans l'intervalle d'environ 3,0 à 4,0, en opérant de préférence dans un système tampon, par exemple un tampon acide acétique/acétate d'ammonium.

3. Procédé selon les revendications 1 et 2, caractérisé en ce que l'on ajoute au mélange de réaction, avant l'isolement du produit de l'étape d'hydrolyse, un composé carbonylé non stéroïde, et on utilise de préférence des cétones ou aldéhydes et on préfère des composés aliphatiques ou cycloaliphatiques correspondants jusqu'en $C_{10}$ qui peuvent aussi être oléfiniques.

4. Procédé selon les revendications 1 à 3, caractérisé en ce que la quantité du composé carbonylé ajouté est comprise dans l'intervalle de 0,01 à 10 équivalents par équivalent de composé stéroïde.

5. Procédé selon les revendications 1 à 4, caractérisé en ce que l'on ajoute le composé carbonylé non stéroïde au mélange de réaction avant ou pendant l'étape d'hydrolyse.

6. Procédé selon les revendications 1 à 5, caractérisé en ce que l'on opère avec des o-quinones 3-5-dialkyl-substituées, dont les substituants alkyles ont de préférence chacun jusqu'à 10 atomes de carbone, en particulier jusqu'à 5 atomes de carbone.

7. Procédé selon les revendications 1 à 6, caractérisé en ce que la formation des stéroïde-aniles s'effectue à des températures d'environ −10°C à 36°C, de préférence dans des solvants tels que des alcanols, et avantageusement en atmosphère de gaz inerte.

8. Procédé selon les revendications 1 à 7, caractérisé en ce que l'hydrolyse est effectuée à des températures de −10°C à 100°C et de préférence également à l'abri de làoxygène de l'air.

9. Procédé selon les revendications 1 à 8, caractérisé en ce que l'on opère avec la 3,5-di-tert-butyl--o-benzoquinone.

10. Procédé selon les revendications 1 à 9, caractérisé en ce que la réaction est effectuée en une fois sans isolement des stéroïde-aniles se formant comme intermédiaires.